# EUROPEAN PATENT APPLICATION

(11) **EP 1 755 275 A2**
(43) Date of publication of application: **21.02.2007**
(21) Application number: 06016568.5
(22) Date of filing: 08.08.2006
(51) Int. Cl.: H04L 12/26, A63B 24/00

(54) **Methods and apparatus for monitoring quality of service for an exercise machine communication network**

(30) Priority: 08.08.2005 US 199764
(71) Applicant: BRUNSWICK CORPORATION, Lake Forest, Illinois 60045 (US)
(72) Inventor: Padanabhan, Ramanath, Singapore 120518 (SG); Rao, Rajendra, Libertyville Illinois 60048 (US)
(74) Representative: Samson & Partner

(57) **Abstract**

Methods and apparatus for monitoring quality of service for an exercise machine communications network are disclosed. A disclosed example system for monitoring a quality of service for an exercise machine communication network comprises a server and an exercise machine console communicatively coupled to the server via the exercise machine communication network and operatively coupled to an exercise machine. The exercise machine console is configured to communicate exercise machine usage information to the server, and the server is configured to determine network performance associated with the exercise machine network based on the exercise machine usage information.

## Description

### FIELD OF THE DISCLOSURE

This disclosure relates generally to communication networks utilized in fitness center environments and, more particularly, to methods and apparatus for monitoring quality of service for an exercise machine communication network.
BACKGROUND

The ever increasing concern over personal physical health has motivated many people to partake in various types of health and fitness regimens. Most notably, many individuals join health clubs or physical fitness centers and/or purchase home exercise equipment with intentions to exercise regularly and, in some instances, follow a specific exercise regimen. People are often drawn to health clubs because of the variety of available exercise machines, exercise equipment, exercise classes, and exercise instructors. Often, exercise instructors create customized exercise routines to help a client achieve specific fitness goals, such as, for example, to lose weight, gain strength, build muscle, etc. An example routine might include riding a stationary bicycle for 15 minutes as a warm up, a walk on a treadmill configured to provide varying walking speeds and inclines, some strength training, and finally a slow walk on the treadmill to cool-down. An exercise routine is typically written down for the client to facilitate the repetition of the routine on future visits. Alternatively, or additionally, an instructor might provide personal training assistance and walk the client through each step of a customized exercise routine during each visit.
BRIEF DESCRIPTION OF THE DRAWINGS

FIG. 1 is a schematic diagram of an example fitness environment having a plurality of exercise machines communicatively coupled to a server via an exercise machine communication network.

FIG. 2 is example manner of implementing the treadmill of FIG. 1.

FIG. 3 is an example chart illustrating an example relationship between times of day and network delays for the example exercise fitness environment of FIG. 1.

FIG. 4 illustrates an example operation of the example fitness environment of FIG. 1.

FIG. 5 is a flowchart representative of example process that may be performed to implement the example exercise machine console of FIG. 2.

FIG. 6 is a flowchart representative of example process that may be performed to implement the server of FIG. 1.

FIG. 7 is a flowchart representative of example process that may be performed to determine a maximum acceptable network delay for the example fitness environment of FIG 1.

FIG. 8 is a schematic illustration of an example processor platform that may execute the example processes represented by FIGS. 5, 6 and/or 7 to implement the server of FIG. 1 and/or the example machine console of FIG. 2.
DETAILED DESCRIPTION

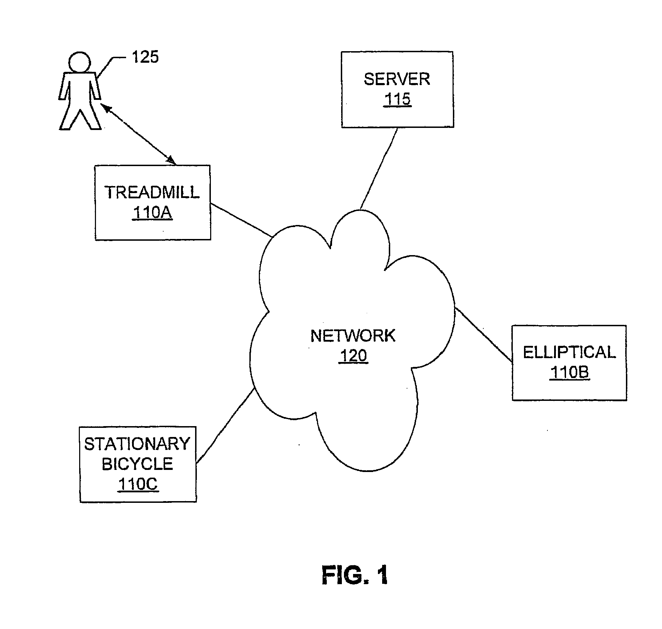

With the increased capability of electronic devices and electronic communications and an increased market value for sophisticated and personalized fitness services, health and fitness centers and clubs are increasingly automating previously manual aspects of their business. FIG. 1 illustrates an example fitness environment that includes a plurality of exercise machines (e.g., a treadmill 110A, an elliptical trainer 110B, and a stationary bicycle 110C) communicatively coupled to a server 115 via an exercise machine communications network 120 (i.e., a network 120).

In the example fitness environment of FIG. 1, the network 120 may be a wireless communications network based, for example, on the Institute of Electrical and Electronics Engineers (IEEE) wireless local area network (WLAN) standard 802.11 g. However, the network 120 could be based on other or additional communication standards and technologies. For instance, WLAN standards IEEE 802.11a, IEEE 802.11b, etc.; wired local area network (LAN) standards IEEE 802.3, IEEE 802.3u, etc.; or other wireless communication technologies, e.g., IEEE 802.15.1 (a.k.a. Bluetooth).

In the illustrated example of FIG. 1, an example user 125 (e.g., a client of the example fitness environment) of one of the plurality of exercise machines 110A-C interacts or communicates with an exercise machine (e.g., the treadmill 110A) to identify themselves and to initiate an exercise routine. The treadmill 110A, in turn, interacts or communicates with the server 115 via the network 120 to obtain an exercise routine for the treadmill 110A. For example, the server 115 may be configured with height and weight information for the example user 125 and with an exercise program for the treadmill 110A customized for the user 125. For instance, a fitness instructor may select for the user 125 a treadmill program comprising a 5 minute warm-up period at 10 miles per hour, a 15 minute period with varying speed and incline, and concluding with a 5 minute cool-down period at 8 miles per hour. The treadmill 110A, having received the customized program information from the server 115 via the network 120 for the user 125, automatically programs and/or configures the treadmill 110A and enables (e.g., starts the treadmill 110A running, prompts the user 125 to provide a start indication, etc.) the customized exercise routine for the example user 125.

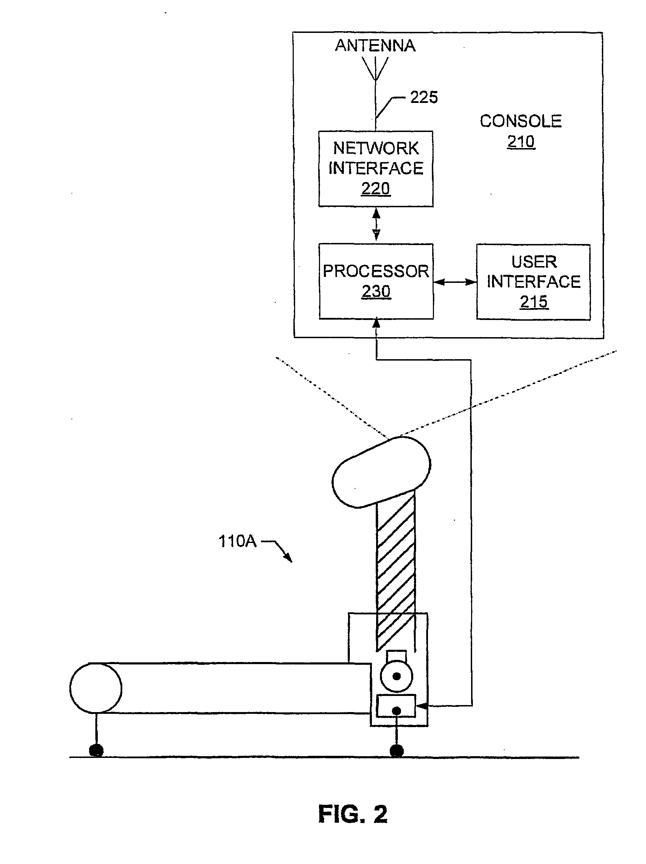

FIG. 2 is an example manner of implementing the example treadmill 110A of FIG. 1. In addition to containing well-known treadmill functionality (e.g., a motor that causes a belt or other walking surface to move beneath a user in a manner that enables the user to walk, run, jog, etc.), the example treadmill 110A or FIG. 2 contains an example exercise machine console 210. As illustrated in FIG. 2, the console 210 is operatively coupled with the well-known treadmill functionality. For instance, the console 210 can control the speed of the motor that moves the walking surface and adjust an incline angle of the walking surface.

To enable the user 125 to interact with the example treadmill 110A of FIG. 2, the example console 210 includes an example user interface 215 that, among other things, provides indications to the user 125 and receives status information from the treadmill 110A. The example user interface 215 includes buttons to enable the user 125 to enter a code identifying the user 125 (e.g., 537) and a button to request a start of a customized exercise program.

In another example, the user interface 215 may utilizes an electronic card reader configured to read an identification card carried by the user 125. For instance, the user 125 may pass an edge of the card through the electronic card reader to transfer identification information to the treadmill 110A. The example user interface 215 could include a liquid crystal display (LCD) and/or light emitting diodes (LEDs) to display a pictorial or graphical representation of the exercise program and to indicate where the user 125 currently is within the exercise program. The user interface 215 may also employ any of a variety of other interface technologies, such as, for example, a touch screen, membrane switches, etc.

The example console 210 of FIG. 2 also includes a network interface 220 and an antenna 225 to enable the console 210 to communicate with the server 115 via the network 120. The network interface 220 and the antenna 225 may be implemented using one of a variety of standard or customized devices. For example, a TNETW1130™ chip from Texas Instruments, a BCM4317™ chip from Broadcom, etc.

The example console 210 of FIG. 2 further includes a processor 230 configured to communicate with the server 115 via the network interface 220 and the antenna 225, to interact with the user 125 via the user interface 215, and to control the treadmill 110A. In an example implementation, the processor 230 receives from the user interface 215 information identifying a user and a request to initiate an exercise program, provides the identifying information to the server 115 via the network 120, receives an exercise program customized for the user 125 from the server 115, configures and/or programs the treadmill 110A, enables (e.g., starts the treadmill 110A running, prompts the user 125 to provide a start indication, etc.) the customized exercise program, monitors the treadmill 110A, and displays via the user interface 215 progress of the user 125 through the customized exercise program. The example console 210 can also be implemented with other types of exercise machines (e.g., the elliptical trainer 110B, the stationary bicycle 110C, etc.) to provide similar functionality and capabilities to those discussed above for the example treadmill 110A of FIG. 2.

The use of the IEEE 802.11g WLAN standard provides tremendous flexibility in the configuration, operation and maintenance of the example fitness environment of FIG. 1. For example, if an exercise machine (e.g., the stationary bicycle 110C) is relocated within the fitness environment (e.g., a health club) it is not necessary to ensure that a wired connection (e.g., Ethernet cable) is available in the new location. Instead, the stationary bicycle 110C automatically reestablishes connectivity with the network 120 and the server 115 using techniques specified in the IEEE 802.11 g WLAN standard.

However, current WLAN standards (e.g., IEEE 802.11g, IEEE 802.15.1, etc.) can be vulnerable to signal interference. For example, a nearby radio frequency signal transmitter may cause a reduced signal-to-noise ratio (SNR) for the signal path between an exercise machine and the network 120, thereby reducing the achievable communication speed. Further, because the network 120 is a shared communication resource, an increase in traffic (e.g., data being communicated) between the plurality of exercise machines 110A-C and the server 115 may result in communication delays. For example, if the network 120 is overloaded with a large amount of traffic, an exercise machine may not be able to communicate with the server 115 to obtain a customized exercise program or the exercise machine may experience a relatively long delay time before receiving the complete customized exercise program from the server 115.

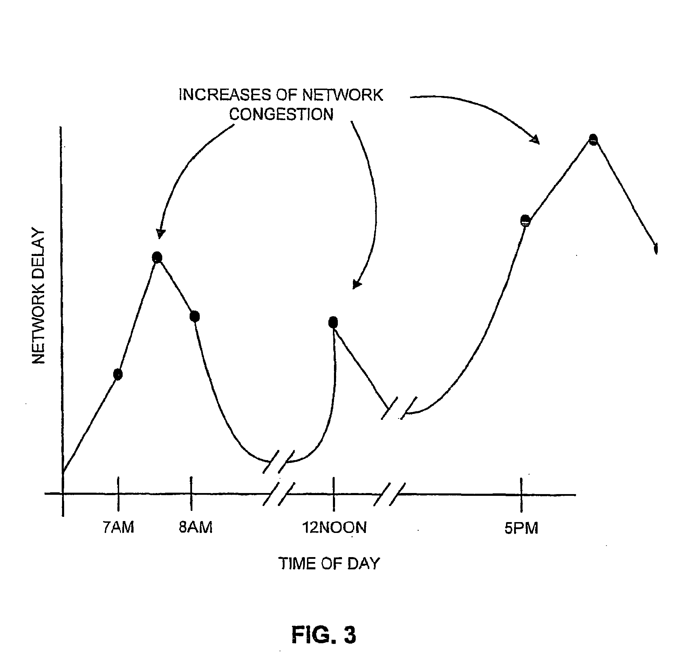

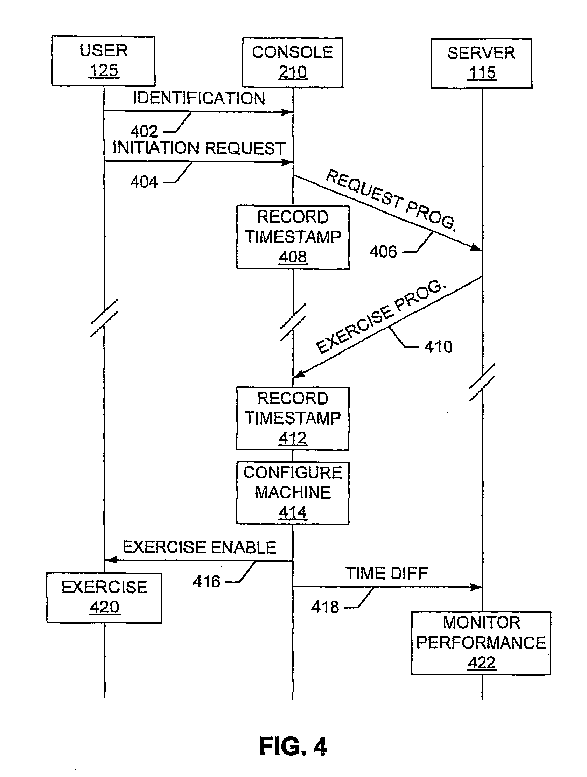

FIG. 3 is an example chart illustrating an example relationship between times of day and network delays (e.g., times between initiated requests to exercise and receipt of corresponding customized exercise programs by an exercise machine). As is well known, the number of clients using a fitness center typically increases during certain periods of a day. For example, before work, lunch hour, after work, etc. As illustrated in FIG. 4, more clients using exercise machines during specific periods of the day resulted in an increase in network traffic, thereby causing an increase in network delays.

If network delays increase sufficiently, clients may begin experiencing a decrease in customer or client satisfaction. That is, clients may become impatient waiting for a customized exercise program to be automatically (as discussed above) received, configured, and enabled. In response, a client may repeatedly press an exercise initiation button assuming the machine is defective, may opt to manually configure the exercise machine, may select another type of machine thus altering their customized and/or preferred exercise routine, or may elect to join another health club where client satisfaction may be higher.

To maintain client satisfaction and to ensure correct and efficient operation of the network 120 and the example fitness environment of FIG. 1, the performance characteristics for the network 120 are monitored. In the illustrated example of FIG. 1, network delay is used as an example performance characteristic. More specifically, elapsed time durations between each request to initiate an exercise program and each receipt of a corresponding customized exercise program are determined and logged. Further, the number of unsuccessful initiation requests, the number of times a user manually configures an exercise machine, and the number of times a user walks away from a machine are also recorded as additional performance characteristics. Other performance characteristics such as, for example, the number of packet retransmissions due to low SNR, etc. could be used instead of or in addition to those mentioned above. Still further, a time of day may be recorded for each exercise request to enable network performance to be tracked as a function of time of day and/or day of the week.

In the illustrated example of FIG. 1, the server 115 notifies an operator of the network 120 (e.g., via pager, cellular telephone, email, etc.) if the performance of the network 120 degrades below a certain pre-determined level. For example, if any or an average network delay exceeds a certain pre-determined threshold, the server 115 alerts the operator. Further, the server 115 may analyze network delay values associated with each exercise machine to identify portions of the fitness environment and/or specific exercise machines experiencing large network delays. In response to an alert, the operator may take an appropriate corrective action. For example, the operator may relocate one or more exercise machines, locate an offending radio frequency transmitting device, etc.

FIG. 4 illustrates an example operation of the example fitness environment of FIG. 1. In the example of FIG. 4, the user 125 provides identifying information to the console 210 (line 402) and initiates a request to start a customized exercise program (line 404). In response to the initiation request (line 404), the console 210 sends a request for a customized exercise program to the server 115 via the network 120 (line 406) and records a time stamp corresponding to receipt of the exercise initiation request (box 408).

The request for the customized exercise program (line 406) is received by the server 115 some time later, where the amount of elapsed time represents the network delay. The server 115 responds to the request for the customized program (line 406) by sending the customized program to the console 210 via the network 120 (line 410). Conveyance of the customized program to the requesting console 210 of the treadmill 110A (line 410) may also be delayed by the network 120. When the console 210 receives the customized program (line 410), the console 210 records a second timestamp (box 412) and programs the treadmill 110A with the customized program (box 414). The console 210 then indicates via the user interface 215 that exercising is enabled (e.g., starts the treadmill 110A running, prompts the user 125 to provide a start indication, etc.) (line 416) and sends a difference between the first and second timestamps to the server 115 (line 418). In response to indication of exercise enablement (line 416), the user 125 exercises or provides a start indication and then exercises (box 420). The server 115 uses the received time difference (line 418) to monitor the performance of the network 120 (box 422).

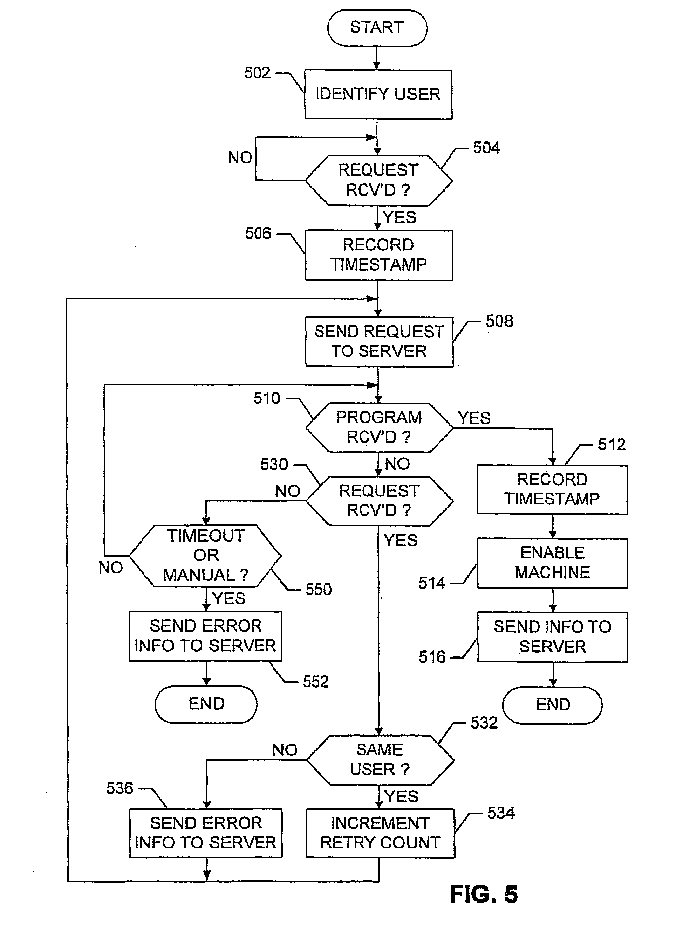

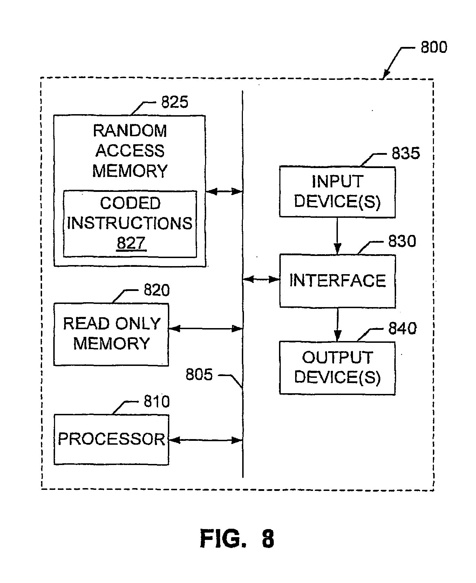

FIGS. 5, 6 and 7 illustrate flowcharts representative of example process that may be performed to implement the example operation of FIG. 4, the console 210, the example server 115 and/or the example fitness environment of FIG. 1. The example processes of FIGS. 5-7 may be executed by a processor, a controller and/or any other suitable processing device. For example, the example processes of FIGS. 5-7 may be embodied in coded instructions stored on a tangible medium such as a flash memory, or random access memory (RAM) associated with the processor 810 shown in the example processor platform 800 and discussed below in conjunction with FIG. 8. Alternatively, some or all of the example processes of FIGS. 5-7 may be implemented using an application specific integrated circuit (ASIC), a programmable logic device (PLD), a field programmable logic device (FPLD), discrete logic, hardware, etc. Also, some or all of the processes of FIGS. 5-7 may be implemented manually or as combinations of any of the foregoing techniques. Further, although the example processes of FIGS. 5-7 are described with reference to the flowcharts of FIG. 5-7, persons of ordinary skill in the art will readily appreciate that many other methods of implementing the console 210, the server 115 and/or the example fitness environment of FIG. 1 may be employed. For example, the order of execution of the blocks may be changed, and/or some of the blocks described may be changed, eliminated, or combined.

Turning to FIG. 5, an example process that may be performed to implement the example console 210 of FIG. 2. The example process of FIG. 5 begins when the user 125 starts a new interaction with the console 210. The console 210 via the user interface 215 receives information identifying the user 125 (block 502). If a request to initiate exercise is not received (block 504), the console 210 remains at block 504 to await an exercise initiation request. If a request to initiate exercise is received (block 504), the console 210 records a timestamp corresponding to receipt of the initiation request (block 506) and sends a request for a customized exercise program to the server 115 via the network 120 (block 508). The request may include, for example, the information identifying the user 125 and the console 210 or the exercise machine associated with the console 210.

At block 510, the console 210 determines if the customized exercise program has been received. If the customized program is received (block 510), the console 210 records another timestamp corresponding to receipt of the initiation request (block 512), configures and enables the exercise machine (e.g., starts the elliptical trainer 110B running) (block 514), sends information indicative of network performance to the server 115 (block 516) and ends execution of the example process of FIG. 5.

In the process of FIG. 5, the console 210 sends a difference between the two recorded timestamps and the number of initiation requests to the server 115. Alternatively, the console 210 could send the two timestamps to the server 115. Further, each exchanged message could include information sufficient to allow the server 115 to determine and record timestamps. For example, the initiation request sent in block 508 could include a time indicative of when the request was made by the user 125 and the console 210 could send a message to the server 115 indicating the time at which the customized program was received by the console 210 (block 516). Thus, each of the two exchanges conveys enough information to determine a network delay. For instance, the server 115 records the time when a message is sent to the console 210, the console 210 records when the messages is received and sends the arrival time of the message to the server 115, and the server 115 computes a difference to determine the network delay.

Returning to block 510, if the customized exercise program has not been received, the console 210 determines if an additional exercise request has been made by the user 125 (e.g., by the user re-swiping an identification card) (block 530). If an additional request has been made (block 530), the console 210 determines if the same user is making the request (block 532) by, for example, prompting the user to re-enter identifying information or using the information obtained from a swipe of an identification card. If the request was made by the same user (block 532), the console 210 increments a count of initiation requests (block 534) and returns to block 508 to send another request for the customized program to the server 115. If the request was made by a different user (block 532), the console 210 sends error information to the server 115 indicating that the previous user abandoned using the exercise machine (block 536) and returns to block 508 to send a request for a customized exercise program to the server 115.

Returning to block 530, if another initiation request has not been received, the console 210 determines if either a timeout has occurred or if the user 125 has manually configured an exercise program (e.g., gave up waiting for the console 210 to automatically configure and enable the exercise machine) (block 550). If neither has occurred (block 550), the console 210 returns to block 510 to determine if the customized exercise program has been received. If either a timeout or manual configuration has occurred (block 550) the console 210 sends error information to the server 115 and ends executing the example process of FIG. 5. Example error information includes information associated with the cause of the failure (e.g., a timeout or manual configuration), number or initiation request retries, etc.

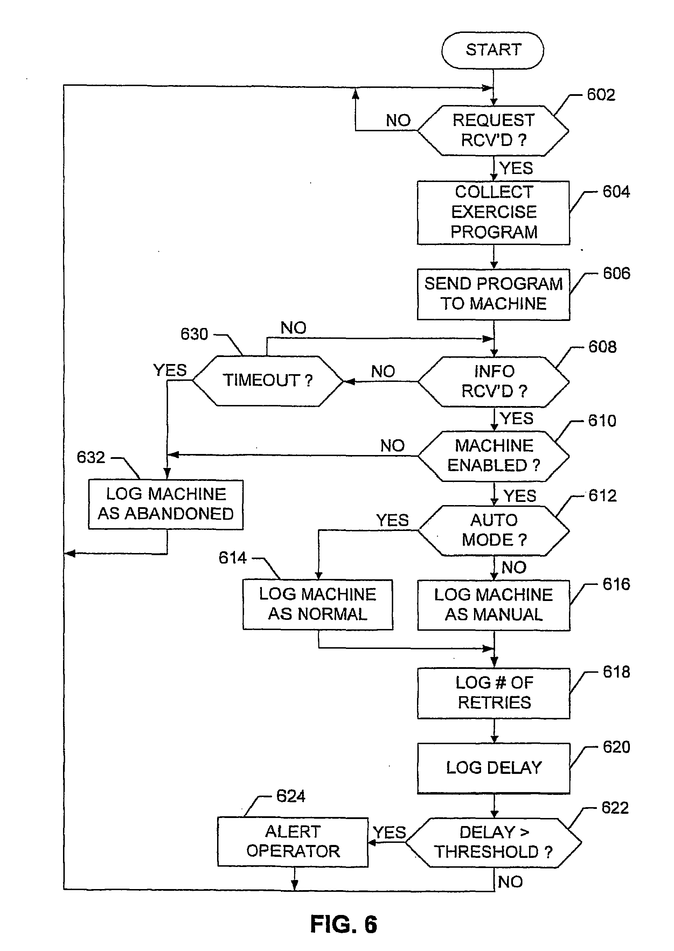

Turning to FIG. 6, an example process that may be performed to implement the example server 115 of FIG. 1. The example process of FIG. 6 begins when the server 115 initiates processing and proceeds indefinitely until terminated by, for example, a processor restart, an operator, etc. If a request for a customized exercise program is not received from an exercise machine (block 602), the server 115 remains at block 602 awaiting a request. If a request is received (block 602), the server 115 collects the customized exercise program corresponding to the user 125 and the exercise machine being used by the user 125 from a database (block 604) and sends the customized exercise program to the exercise machine (block 606).

The server 115 then determines if network delay information (e.g., a difference between timestamps recorded by the console 210) has been received (block 608). If the delay information has been received (block 608), the server 115 determines if the exercise machine is enabled and/or running (e.g., executing either the automatically configured customized program or a manual program) (block 610). If the exercise machine is enabled and/or running (block 610), the server 115 determines if the machine was automatically configured by the console 210 using the customized program received from the server 115 and enabled by the console 210 (block 612). If the machine was automatically configured (block 612), the server 115 logs the machine as running in normal mode (i.e., successful automatic configuration) (block 614), otherwise the server 115 logs the machine as running is manual mode (e.g., the user 125 got tired of waiting and manually configured the exercise machine) (block 616).

The server 115 then records the number of initiation requests or retries made by the user 125 and reported by the console 210 before the customized program was received and started by the console 210 (block 618) and logs the network delay information received from the console 210 (block 620). In the example process of FIG. 6, if the network delay information received from the console 210 exceeds a threshold (block 622), the server 115 sends an alert to an operator (block 624). If the delay does not exceed the threshold (block 622), the server 115 returns to block 602 to await another request from an exercise machine for a customized exercise program.

When an operator is alerted that a network delay for an automatic configuration exceeds the threshold, the operator can take any of a variety of corrective actions. For example, the operator may determine if the exercise machine is malfunctioning, relocate the exercise machine, etc. If the operator is alerted that the network delays for multiple exercise machines exceed the threshold, then the operator could, for example, determine if a radio frequency transmitter is causing interference, upgrade the network 120, etc.

Returning to block 610, if the exercise machine is not enabled and/or running, the server 115 logs the machine as abandoned (e.g., the user 125 gave up on waiting for the customized exercise program to start) (block 632), and the server 115 returns to block 602 to await another request from an exercise machine for a customized exercise program.

Returning to block 608, if the network delay information has not been received, the server 115 determines if a timeout has occurred (block 630). If a timeout has occurred (block 630), the server 115 logs the exercise machine as abandoned (i.e., the user 125 gave up on waiting for the customized exercise program to start) (block 632), and the server 115 returns to block 602 to await another request from an exercise machine for a customized exercise program. If a timeout has not occurred (block 630), the server 115 returns to block 608 to continue waiting for the network delay information.

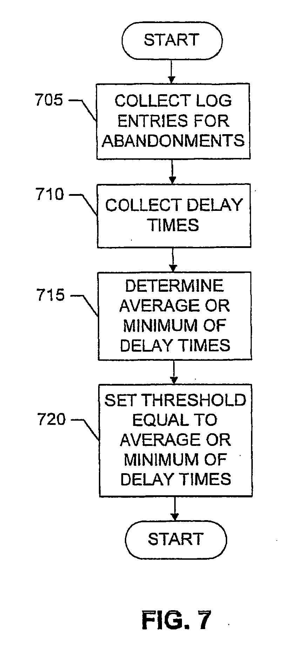

Turning to FIG. 7, an example process that may be performed to determine a maximum acceptable network delay for the example fitness environment of FIG. 1. The example process of FIG. 7 begins with the server 115 identifying all entries in the log corresponding to, for example, all exercise initiation requests that resulted in a user ceasing to wait for the machine to start (i.e., logged as abandoned) (block 705). For all of the identified log entries, the server 115 collects the network delay times (block 710) and then determines, for example, an average or minimum of the collected network delay times (block 715). Finally, the server 115 sets the threshold equal to the determined value (block 720) and ends executing the example process of FIG. 7.

It will be understood that the example network 120 of FIG. 1 could be constructed using a network topology different from the client-server topology discussed above. For example, the treadmill 110A, the elliptical trainer 110B and the stationary bicycle 110C could create a web or mesh network with one of them (e.g., the treadmill 110A) serving as a bridge to the server 115 for the others (e.g., the elliptical trainer 110B, the stationary bicycle 110C). In particular, the elliptical trainer 110B and the stationary bicycle 110C could establish communications with the treadmill 110A which relays communications on their behalf to and from the server 115. Further, the performance characteristics of the network 120 may be enhanced to include each of the plurality of links comprising the network 120. For instance, a link between the elliptical trainer 110B and the treadmill 110A, a link between the stationary bicycle 110C and the treadmill 110A, and a link between the treadmill 110A and the server 115, etc.

FIG. 8 is a schematic diagram of an example processor platform 800 capable of executing the example operation illustrated in FIG. 4 and/or the example processes of FIGS. 5-7 to implement the console 210, the server 115 and/or the example fitness environment of FIG. 1. For example, the processor platform 800 can be implemented by one or more general purpose microprocessors, microcontrollers, etc.

The processor platform 800 of the example of FIG. 8 includes a general purpose programmable processor 810. The processor 810 executes coded instructions 827 present in main memory of the processor 810 (e.g., within a RAM 825). The processor 810 may be any type of processing unit, such as a microprocessor from the Intel®, AMD®, or SUN® families of microprocessors. The processor 810 may implement, among other things, the console 210, the server 115 and/or the example fitness environment of FIG. 1 by, for example, executing the example operation of FIG. 4 and/or the example processes of FIGS. 5-7.

The processor 810 is in communication with the main memory (including a read only memory (ROM) 820 and the RAM 825) via a bus 805. The RAM 825 may be implemented by Synchronous Dynamic Random Access Memory (SDRAM), Dynamic DRAM, and/or any other type of RAM device. The ROM 820 may be implemented by flash memory and/or any other desired type of memory device. Access to the memory 820 and 825 is typically controlled by a memory controller (not shown) in a conventional manner.

The processor platform 800 also includes a conventional interface circuit 830. The interface circuit 830 may be implemented by any type of well-known interface standard, such as an external memory interface, serial port, general purpose input/output, etc.

One or more input devices 835 and one or more output devices 840 are connected to the interface circuit 830. The input devices 835 and output devices 840 may be used to implement interfaces between the console 210 and an exercise machine (e.g., the treadmill 110A), the processor 230 and the network interface 220, and/or the user interface 215.

Of course, persons of ordinary skill in the art will recognize that the order, size, and proportions of the memory illustrated in the example systems may vary. Additionally, although this patent discloses example systems including, among other components, software or firmware executed on hardware, it should be noted that such systems are merely illustrative and should not be considered as limiting. For example, it is contemplated that any or all of these hardware and software components could be embodied exclusively in hardware, exclusively in software, exclusively in firmware or in some combination of hardware, firmware and/or software. Accordingly, persons of ordinary skill in the art will readily appreciate that the above described examples are not the only way to implement such systems.

At least some of the above described example methods and/or apparatus are implemented by one or more software and/or firmware programs running on a computer processor. However, dedicated hardware implementations including, but not limited to, an ASIC, programmable logic arrays and other hardware devices can likewise be constructed to implement some or all of the example methods and/or apparatus described herein, either in whole or in part. Furthermore, alternative software implementations including, but not limited to, distributed processing or component/object distributed processing, parallel processing, or virtual machine processing can also be constructed to implement the example methods and/or apparatus described herein.

It should also be noted that the example software and/or firmware implementations described herein are optionally stored on a tangible storage medium, such as: a magnetic medium (e.g., a disk or tape); a magnetooptical or optical medium such as a disk; or a solid state medium such as a memory card or other package that houses one or more read-only (nonvolatile) memories, random access memories, or other re-writable (volatile) memories; or a signal containing computer instructions. A digital file attachment to e-mail or other self-contained information archive or set of archives is considered a distribution medium equivalent to a tangible storage medium. Accordingly, the example software and/or firmware described herein can be stored on a tangible storage medium or distribution medium such as those described above or equivalents and successor media.

To the extent the above specification describes example components and functions with reference to particular devices, standards and/or protocols, it is understood that the teachings of the invention are not limited to such devices, standards and/or protocols. For instance, the IEEE 802.11g and IEEE 802.3z standards represent examples of the current state of the art. Such standards are periodically superseded by faster or more efficient equivalents having the same general functionality. Accordingly, replacement devices, standards and/or protocols having the same functions are equivalents which are contemplated by the teachings of the invention are intended to be included within the scope of the accompanying claims.

The teachings of the invention contemplate one or more machine readable mediums containing instructions, or receiving and executing instructions from a propagated signal so that, for example, a device connected to a network environment can send or receive voice, video or data, and communicate over the network using the instructions. Such a device can be implemented by any electronic device that provides voice, video or data communication, such as a telephone, a cordless telephone, a mobile phone, a cellular telephone, a Personal Digital Assistant (PDA), a set-top box, a computer, and/or a server.

Although certain example methods, apparatus and articles of manufacture have been described herein, the scope of coverage of this patent is not limited thereto. On the contrary, this patent covers all methods, apparatus and articles of manufacture fairly falling within the scope of the appended claims either literally or under the doctrine of equivalents.

## Claims

1. A method of monitoring quality of service for an exercise machine communication network, comprising:
monitoring usage of a plurality of exercise machines to generate exercise machine usage information; and
determining a performance characteristic of the exercise machine communications network based on the exercise machine usage information.

2. A method as defined in claim 1, further comprising communicating the exercise machine usage information to a server configured to determine the performance characteristic.

3. A method as defined in claim 1, wherein monitoring the usage of the plurality of exercise machines includes monitoring exercise initiation requests and exercise machine enablements.

4. A method as defined in claim 1, wherein monitoring the usage of the plurality of exercise machines includes determining when at least one of the plurality of exercise machines is operating in at least one of an automatic mode or a manual mode.

5. A method as defined in claim 1, wherein monitoring the usage of the plurality of exercise machines includes logging a number of exercise initiation requests associated with an exercise machine enablement.

6. A method as defined in claim 1, wherein the performance characteristic is indicative of user satisfaction of the exercise machine communications network.

7. A method as defined in claim 1, wherein the performance characteristic is indicative of at least one of network congestion or network delay.

8. A method as defined in claim 1, wherein determining the performance characteristic comprises:
generating a first plurality of timestamps associated with respective ones of a plurality of exercise initiation requests;
generating a second plurality of timestamps associated with respective ones of a plurality of exercise machine enablements; and
determining the performance characteristic based differences between the first and the second plurality of timestamps.

9. A method as defined in claim 1, further comprising alerting an operator based on the performance characteristic.

10. A method as defined in claim 9, wherein the operator is alerted if the performance characteristic exceeds a pre-determined threshold.

11. A method of monitoring quality of service for an exercise machine communication network, comprising:
recording a first occurrence time of a first event;
receiving a second occurrence time of a second event; and
determining a performance characteristic of the exercise machine communication network based on the first and second occurrence times.

12. A method as defined in claim 11, wherein the first event is an exercise initiation request and the second event is an exercise machine enablement.

13. A method as defined in claim 11, further comprising recording a time of day associated with the first or the second occurrence time.

14. A method as defined in claim 11, wherein the performance characteristic is a measure of network performance.

15. A method as defined in claim 11, wherein the performance characteristic is based on a difference between the first and second occurrence times.

16. A method as defined in claim 11, wherein the performance characteristic is an amount of time a user waits for an exercise machine enablement.

17. A system for monitoring quality of service for an exercise machine communication network, comprising:
a server; and
an exercise machine console communicatively coupled to the server via the exercise machine communication network and operatively coupled to an exercise machine, wherein the exercise machine console is configured to communicate exercise machine usage information to the server, and wherein the server is configured to determine network performance associated with the exercise machine network based on the exercise machine usage information.

18. A system as defined in claim 17, wherein the exercise machine console comprises:
a network device communicatively coupled to the server; and
a processor operatively coupled to the exercise machine and communicatively coupled to the network device, wherein the processor is configured to record the exercise machine usage information and communicate the exercise machine usage information to the server via the network device.

19. A system as defined in claim 17, wherein the network performance is indicative of a delay in the start of exercise by a user of the exercise machine.

20. A system as defined in claim 17, wherein the server notifies an operator if the network performance falls below a pre-determined threshold.

21. A system as defined in claim 20, wherein the pre-determined threshold represents a maximum delay acceptable to a user of the exercise machine.

22. A system as defined in claim 17, wherein the exercise machine usage information includes an exercise initiation request associated with a request to use the exercise machine and an exercise machine enablement associated with receiving exercise information at the exercise machine from the server in response to the exercise initiation request.

23. A system as defined in claim 22, wherein the exercise machine usage information includes a time of day associated with the exercise initiation request or the exercise machine enablement and wherein the network performance is associated with the time of day.

24. A system as defined in claim 22, wherein the exercise machine usage information includes a time difference between the exercise initiation request and exercise machine enablement.
